Europäisches Patentamt

European Patent Office

Office européen des brevets

(19)

(11) Publication number: **0 014 998**

A1

(12)

# EUROPEAN PATENT APPLICATION

(21) Application number: 80100839.2

(22) Date of filing: 20.02.80

(51) Int. Cl.³: **C 07 C 85/08**
C 07 C 87/62, C 07 C 87/58
C 07 C 87/66, B 01 J 27/04

(30) Priority: 21.02.79 US 13341

(43) Date of publication of application:
03.09.80 Bulletin 80/18

(84) Designated Contracting States:
BE DE FR GB IT

(71) Applicant: UNIROYAL, INC.
1230 Avenue of the Americas Rockefeller Center
New York, New York 10020(US)

(72) Inventor: Malz, Russel E. Jr.
261 Wedgewood Drive
Naugatuck, New Haven Connecticut(US)

(72) Inventor: Greenfield, Harold
85 Crestview Drive
Watertown, Litchfield Connecticut(US)

(72) Inventor: Wheeler, Edward Lockwood
126 Claxton Avenue
Watertown, Litchfield(US)

(74) Representative: Bühling, Gerhard, Dipl.-Chem.
Patentanwaltsbüro Tiedtke-Bühling-Kinne
Grupe-Pellmann Bavariaring 4
D-8000 München 2(DE)

(54) Preparation of tertiary aromatic amines by reductive alkylation of diaryl secondary amines with ketones.

(57) Tertiary aromatic amines are prepared by the reductive alkylation of diaryl secondary amines with ketones in the presence of hydrogen and a heterogenous hydrogenation catalyst.

EP 0 014 998 A1

The present invention relates to the use of heterogeneous hydrogenation catalysts for the reductive alkylation of diaryl secondary amines with ketones in the presence of hydrogen to produce N-alkyldiaryl tertiary amines. Such tertiary aromatic amines and their derivatives are useful as fabric softeners, asphalt emulsifiers, petroleum additives, floatation agents, chemical intermediates, and as stabilizers for polymers, rubbers, gasoline, and synthetic lubricants.

The present invention relates to a method for preparing a compound having the formula A-NR-B wherein A is phenyl, naphthyl, phenyl substituted with up to three groups, which may be the same or different and may be chlorine, bromine, linear or branched $C_1$-$C_{12}$ alkyl, or naphthyl substituted with linear or branched $C_1$-$C_{12}$ alkyl; B may be the same as or different from A and has the meanings of A, provided that B does not have more than three substituents including an amino moiety, or B is -$C_6H_4NR^1R^2$, wherein $R^1$ and $R^2$ may be the same or different and are hydrogen, provided that at least one of $R^1$ and $R^2$ is other than hydrogen, linear or branched $C_1$-$C_{18}$ alkyl, $C_5$-$C_6$ cycloalkyl, or $C_6$-$C_{10}$ aryl, provided that only one of $R^1$ and $R^2$ is naphthyl, and R is -$CHR^4R^5$ wherein $R^4$ and $R^5$ may be the same or different and are linear or branched $C_1$-$C_{18}$ alkyl or $R^4$ and $R^5$ combined are tetramethylene or pentamethylene; by reacting a compound of the formula A-NH-B, wherein A and B have the meanings defined above, except that $R^1$ or $R^2$ or both may be hydrogen, with a compound having the formula $OCR^4R^5$ wherein $R^4$ and $R^5$ are as defined above; in the presence of an heterogeneous catalyst selected from metals of groups 1b, 6b, 7b and 8 or their oxides or sulfides, and copper chromite, except for silver, gold, technetium, manganese and chromium and their oxides or sulfides, in the presence of hydrogen. The present invention also relates to compounds produced by the process of the present invention and to certain novel compounds of the formula A-NR-B wherein A, R and B are as defined above.

In a preferred method of the present invention the starting materials are compounds of the formula A-NH-B wherein A is phenyl or naphthyl; B is phenyl, naphthyl or $-C_6H_4NR^1R^2$ wherein $R^1$ is $C_1-C_3$ alkyl, phenyl or naphthyl, and $R^2$ is hydrogen, linear or branched $C_1-C_7$ alkyl or cyclohexyl; and R is $-CHR^4R^5$, wherein $R^4$ is methyl and $R^5$ is $C_1-C_5$ alkyl or $R^4$ and $R^5$ combined are pentamethylene, or either $R^1$ or $R^2$ or both are hydrogen.

Non-limiting examples of the aryl groups A and B are phenyl and naphthyl. The aryl groups may be substituted on the ring by one or more groups, such as alkyl, phenyl, alkoxy, aryloxy, halo, carboxyl and carboxyl derivatives, thioester, etc., that are not affected by the hydrogenation. Non-limiting examples of diaryl secondary amines useful in the invention are diphenylamine, 3-methyldiphenylamine, N,N'-diphenyl-p-phenylenediamine, N,N'-diphenylbenzidine, N-phenylanthranilic acid, N-phenyl-1-naphthylamine, N-phenyl-2-naphthylamine, N-phenyl-N'-2-naphthyl-p-phenylenediamine, di-2-naphthylamine, N-phenyl-N',N'-dimethyl-p-phenylenediamine, N-phenyl-N'-methyl-N'-isopropyl-p-phenylenediamine and N-phenyl-N,N'-diisopropyl-p-phenylenediamine.

The ketones used for the reductive alkylation may be of any type. Non-limiting examples are dialkyl, alicyclic, alkaryl, and diaryl ketones. Examples of such useful ketones are acetone, 2-butanone, di-n-hexyl ketone, 2,6-dimethyl-3-heptanone, 2,6-dimethyl-4-heptanone, 3,5-dimethyl-4-heptanone, 2,4-dimethyl-3-pentanone, 4,4-dimethyl-2-pentanone, 2-heptanone, 3-heptanone, 4-heptanone, 2-hexanone, 3-hexanone, mesityl oxide, 4-methyl-3-heptanone, 5-methyl-2-hexanone, 3-methyl-2-pentanone, 10-nona-decanone, 2-nonanone, 5-nonanone, 2-octanone, 3-octanone, 4-octanone, 2-pentanone, 3-pentanone, 2-undecanone, 6-undecanone, isophorone, benzophenone, 4-methoxybenzophenone, 2-methylbenzophenone, 4-methylbenzophenone, 2,4,5-trimethoxybenzophenone, 2,4,6-trimethoxybenzophenone, acetophenone, 2-chloroacetophenone, 4-chloroacetophenone, 2-methoxyacetophenone, 3-methoxyacetophenone, 4-methoxyacetophenone, 2-methylaceto-phenone, 3-methylacetophenone, 4-methylacetophenone, cyclohexanone, cyclopentanone and 3-methylcyclohexanone.

The hydrogenation process of the present invention may be carried out in the absence of any solvent medium or in the presence of an inert organic solvent, including, by way of non-limiting example, aliphatic or cycloaliphatic alcohols, such as methyl alcohol, ethyl alcohol, n-propyl alcohol, isopropyl alcohol, n-butyl alcohol, isobutyl alcohol, sec-butyl alcohol, tert-butyl alcohol, n-amyl alcohol, isoamyl alcohol, tert-amyl alcohol, n-hexyl alcohol, cyclohexanol, n-octyl alcohol, 2-ethylhexanol, n-decyl alcohol, n-dodecanol, n-tetradecanol, n-hexadecanol, n-octadecanol, ethylene glycol, propylene glycol, glycerol, etc.; ethers, such as diethyl ether, di-n-propyl ether, di-isopropyl ether, methyl n-butyl ether, ethyl n-butyl ether, di-n-butyl ether, di-n-amyl ether, diisoamyl ether, di-n-hexyl ether, ethylene glycol dimethyl ether, ethylene glycol diethyl ether, ethylene glycol dibutyl ether, 1,3-dioxolane, 1,4-dioxane, tetrahydrofuran; alcohol-ethers, such as diethylene glycol, triethylene glycol, tetraethylene glycol, dipropylene glycol, 2-butoxyethanol, tetrahydrofurfuryl alcohol etc.; esters, such as methyl acetate, ethyl acetate, propyl acetate, isopropyl acetate, butyl acetate, isobutyl acetate, methyl amyl acetate, 2-ethylhexyl acetate, diethyl succinate, etc.; nitriles, such as acetonitrile, propionitrile and butyronitrile; amides, such as formamide, acetamide, propionamide, N,N-dimethylformamide, N,N-diethylformamide, N,N-dimethylacetamide, and N,N-diethylacetamide; hydrocarbons, such as n-hexane, 2-methylpentane, 3-methylpentane, 2,2-dimethylbutane, 2,3-dimethylbutane, cyclohexane, n-heptane, 3-methylhexane, n-octane, 2,2,4-trimethylpentane, 2,3,4-trimethylpentane, n-decane, decalin, benzene, toluene and xylenes.

Non-limiting examples of the heterogeneous hydrogenation catalysts useful for reductive alkylations include metals, such as platinum, palladium, rhodium, ruthenium, osmium, iridium, rhenium, iron, cobalt, nickel and copper; oxides, such as rhenium oxides, copper oxides, and copper-chromium oxides; and sulfides, such as the sulfides of the previously mentioned metals plus the sulfides of molybdenum and tungsten. Preferable catalysts are platinum, palladium, copper-chromium oxides, and the sulfides of platinum, palladium, rhodium, ruthenium, nickel, and cobalt. Most preferred catalysts are often the sulfides of platinum, rhodium and ruthenium.

The catalyst may be prepared in situ or added to the reaction mixture after prior preparation and isolation. Commercial catalysts are available. The catalyst may be prepared and used as a bulk powder or supported on a suitable carrier, such as carbon or alumina. The catalyst may be prepared and used as a powder for liquid phase slurry and for vapor phase fluidized reactions, or as a pellet or granule for liquid or vapor phase fixed bed operations.

The hydrogenation reaction of the present invention may be carried out in either batch or continuous systems, with either tank or pipeline type reactors, in the liquid phase with either slurry or fixed bed catalysts, or in the vapor phase with either fluidized or fixed bed catalysts, operating in a manner well known to those skilled in the art.

The hydrogenation reaction of the present invention may be run at temperatures ranging from room temperature or below, for example, 5°C to 300°C, depending upon the stability of the reactants and products, preferably 50°C to 275°C, most preferably 80°C to 250°C, and at pressures ranging from atmospheric pressure or below to pressures as high as economically practical, for example, up to 70 MPa, preferably from 70 KPa to 35 MPa, more preferably from 0.70 MPa to 17.5 MPa. While the minimum pressure of hydrogen required approaches zero, the reaction would generally be run at atmospheric pressure or greater because it is preferable for reasons of safety to have a positive pressure in the reactor.

## Example 1

### N-Isopropyldiphenylamine by Reductive Alkylation of Diphenylamine with Acetone

A. A mixture of 169 g (1.0 mole) of diphenylamine, 350 ml (4.8 mole) of acetone, and 10.0 g of 5% platinum sulfide on carbon was added to a 1-liter MagneDrive (trademark of Autoclave Engineers, Inc.) autoclave. The vessel was sealed, purged first with nitrogen and then with hydrogen, and pressurized with hydrogen to 3.5 MPa. The reaction mixture was heated with agitation for 8 hours at 115-125°C and 3.5-5.5 MPa. The autoclave was cooled and depressurized. The reaction product was removed and filtered through Celite filter-aid (trademark of Johns-Manville for diatomaceous earth) to remove the catalyst.

A portion of the filtrate was distilled to provide an analytical sample of N-isopropyldiphenylamine, boiling point 151-152°C at 1.6 KPa.

Anal. (Analysis) Calc'd (Calculated) for $C_{15}H_{17}N$: N, 6.64

Found: N, 6.68

6

0014998

B. Example 1A was repeated with 10.0 g of 5% rhodium sulfide on carbon for 24 hours at 220-235°C and 4.9-8.7 MPa. The reaction product was treated as in Example 1A and was shown by glpc (gas liquid phase chromatography) analysis to contain N-isopropyldiphenylamine.

## Example 2

### N-sec-Butyldiphenylamine by Reductive Alkylation of Diphenylamine with Methyl Ethyl Ketone

A mixture of 169 g (1.0 mole) of diphenylamine, 350 ml (3.9 mole) of methyl ethyl ketone, and 10.0 g of 5% platinum sulfide on carbon was added to a 1-liter MagneDrive autoclave. The vessel was sealed, purged first with nitrogen and then with hydrogen, and pressurized with hydrogen to 4.1 MPa. The reaction mixture was heated with agitation for 6 hours at 115-125°C and 3.5-5.5 MPa. The autoclave was cooled and depressurized. The reaction product was removed and filtered through Celite filter-aid to remove the catalyst. The solvent was removed using a rotary evaporator on a steam bath under reduced pressure. Elution chromatography of a portion of the residue on a silica gel column provided an analytical sample of liquid N-sec-butyldiphenylamine.

Anal. Calc'd for $C_{16}H_{19}N$: C, 85.27; H, 8.52; N, 6.21.

Found: C, 85.56; H, 8.51; N, 6.22

Example 3

## N-1,3-Dimethylbutyldiphenylamine by Reductive Alkylation of Diphenylamine with Methyl Isobutyl Ketone

A. A mixture of 169 g (1.0 mole) of diphenylamine, 350 ml (2.8 mole) of methyl isobutyl ketone, and 10.0 g of 5% platinum sulfide on carbon was added to a 1-liter MagneDrive autoclave. The vessel was sealed, purged first with nitrogen and then with hydrogen, and pressurized with hydrogen to 4.1 MPa. The reaction mixture was heated with agitation for 9.8 hours at 230-240°C and 6.2-8.3 MPa. The autoclave was cooled and depressurized. The reaction product was removed and filtered through Celite filter-aid to remove the catalyst. The solvent was removed using a rotary evaporator on a steam bath under reduced pressure. Elution chromatography of a portion of the residue on a silica gel column provided an analytical sample of liquid N-1,3-dimethylbutyldiphenyl-amine.

Anal. Calc'd for $C_{18}H_{23}N$:  C, 85.32; H, 9.16; N, 5.52 Found:  C, 85.26; H, 9.01; N, 5.85

A second portion of the residue was separated by elution chromatography on a silica gel column into a first fraction consisting of the equivalent of 28 g of N-1,3-dimethylbutyldiphenylamine, identified by tlc (thin layer chromatography); a second fraction consisting of the equivalent of 18 g and shown by tlc to be mostly diphenylamine with a trace of N-1,3-dimethylbutyldiphenylamine; and a third fraction consisting of the equivalent of 129 g of diphenylamine, identified by tlc and melting point. The yields were estimated to be 87% of recovered diphenylamine and 11% of N-1,3-di-methylbutyldiphenylamine (all yields referred to herein are based on the molar quantity of the limiting reagent).

B.  Example 3A was repeated with 10.0 g of 5% rhodium sulfide on carbon for 18.8 hours at 230-240°C and 6.2-8.3 MPa. The catalyst was removed as in Example 3A and the filtrate was concentrated using a rotary evaporator on a steam bath under reduced pressure.  A portion of the concentrated filtrate was separated by elution chromatography on a silica gel column into a first fraction consisting of the equivalent of 81 g (32% yield) of N-1,3-dimethylbutyldiphenylamine, identified by tlc; and a second fraction equivalent to 122 g (72% yield) of recovered diphenylamine, identified by tlc and melting point.

## Example 4

### N-1,4-Dimethylpentyldiphenylamine by Reductive Alkylation of Diphenylamine with Methyl Isoamyl Ketone

A mixture of 169 g (1.0 mole) of diphenylamine, 350 ml (2.5 mole) of methyl isoamyl ketone, and 9.8 g of 5% rhodium sulfide on carbon was added to a 1-liter MagneDrive autoclave.  The vessel was sealed, purged first with nitrogen and then with hydrogen, and pressurized with hydrogen to 4.1 MPa.  The reaction mixture was heated with agitation for 22 hours at 185-190°C and 3.5-5.5 MPa.  The autoclave was cooled and depressurized.  The reaction mixture was removed and filtered through Celite filter-aid to remove the catalyst.  The solvent was removed using a rotary evaporator on a steam bath under reduced pressure.  Elution chromatography of a portion of the residue on a silica gel column indicated a recovery of 76 g (45% yield) of diphenylamine, and the formation of 132 g (49% yield) of liquid N-1,4-dimethylpentyldiphenylamine.

Anal.  Calc'd for $C_{19}H_{25}N$:  C, 85.34; H, 9.42; N, 5.24 Found:  C, 85.14; H, 9.37; N, 5.37

## N-Cyclohexyldiphenylamine by Reductive Alkylation of Diphenylamine with Cyclohexanone

A. A mixture of 169 g (1.0 mole) of diphenylamine, 350 ml (3.4 mole) of cyclohexanone, and 10.0 g of 5% platinum sulfide on carbon was added to a 1-liter MagneDrive autoclave. The vessel was sealed, purged first with nitrogen and then with hydrogen and pressurized with hydrogen to 4.8 MPa. The reaction mixture was heated with agitation for 4.8 hours at 75-80°C and 3.5-5.5 MPa. The autoclave was cooled and depressurized. The reaction product was removed and filtered through Celite filter-aid to remove the catalyst.

A portion of the filtrate was distilled. The fraction boiling at 172-205°C at 1.2 KPa was elution chromatographed on a silica gel column to yield N-cyclohexylidphenylamine, melting at 73.5-74.5°C, that did not have any N-H bands in its infrared spectrum.

Glpc analysis of the undistilled filtrate indicated the presence of 123 g (73% yield) of recovered diphenylamine, 47 g (19% yield, 70% based on conversion, that is, based on converted diphenylamine) of N-cyclohexyldiphenylamine, and showed that about 59% of the cyclohexanone was unreacted and about 25% converted to cyclohexanol.

B. Example 5A was repeated with 10.0 g of 5% rhodium sulfide on carbon for 19 hours at 150°C and 3.5-5.5 MPa. Treatment and analysis of the reaction product as in Example 5A indicated the presence of 67.6 g (40% yield) of recovered diphenylamine, 146 g (58% yield, 97% based on conversion) of N-cyclohexyldiphenylamine, and showed that about 71% of the cyclohexanone was unreacted and that about 11% was converted to cyclohexanol.

BAD ORIGINAL

C. Example 5A was repeated with 10.0 g of 5% ruthenium sulfide on carbon for 0.7 hour at 100-105°C and 3.5-5.5 MPa. Treatment and analysis of the reaction product as in Example 5A indicated the presence of 150 g (89% yield) of recovered diphenylamine, 12 g (5% yield, 89% based on conversion) of N-cyclohexyldiphenylamine, and showed that about 68% of the cyclohexanone was unreacted and about 32% converted to cyclohexanol.

D. Bulk ruthenium sulfide was prepared by passing hydrogen sulfide gas through a solution of 10.0 g of hydrated ruthenium trichloride until precipitation of the black sulfide appeared complete. The precipitate was filtered and washed successively with water, 2-propanol and cyclohexanone. Example 5A was repeated with an estimated 5.5 g of this ruthenium sulfide catalyst for 5.5 hours at 110-120°C and 3.5-5.5 MPa. Treatment and analysis of the reaction product as in Example 5A indicated the presence of 90 g (53% yield) of recovered diphenylamine, and 92 g (37% yield, 79% based on conversion) of N-cyclohexyldiphenylamine, and showed that about 71% of the cyclohexanone was unreacted and about 5% was converted to cyclohexanol.

E. Bulk palladium sulfide was prepared by passing hydrogen sulfide gas through a solution of 10.0 g of palladium chloride dihydrate in 1 liter of 0.3 N hydrochloric acid until precipitation of the black sulfide appeared to be complete. The precipitate was filtered and was washed successively with water, 2-propanol and cyclohexanone. Example 5A was repeated with an estimated 6.5 g of this palladium sulfide catalyst for 10.5 hours at 150-160°C and 3.5-5.5 MPa. Treatment and analysis of the reaction product as in Example 5A indicated the presence of 86 g (51% yield) of recovered diphenylamine, and 105 g (42% yield, 86% based on conversion) of N-cyclohexyldiphenylamine, and showed that about 59% of the cyclohexanone was unreacted and about 16% was converted to cyclohexanol.

F. Bulk nickel sulfide was prepared by adding a solution of 25.6 g of sodium sulfide monohydrate and 7.1 g of sulfur in 166 ml of water, with stirring, to a solution of 26.4 g of nickel chloride hexahydrate in 189 ml of water. The black precipitate was filtered and was washed successively with water, 2-propanol and cyclohexanone. Example 5A was repeated with an estimated 10 g of this nickel sulfide catalyst for 4.5 hours at 150-180°C and 3.5-5.5 MPa. Treatment and analysis of the reaction product as in Example 5A indicated the presence of 87 g (51% yield) of recovered diphenylamine, and 4 g (2% yield, 3% based on conversion) of N-cyclohexyldiphenylamine, and showed that about 41% of the cyclohexanone was unreacted and that there was no detectable cyclohexanol.

G. Bulk cobalt sulfide was prepared using 26.4 g of cobalt chloride hexahydrate following the procedure described in Example 5F. Example 5A was repeated with an estimated 10 g of the cobalt sulfide catalyst for 6.8 hours at 230-235°C and 4.8-6.2 KPa. Treatment and analysis of the reaction product as in Example 5A indicated the presence of 104 g (52% yield) of recovered diphenylamine, and 27 g (11% yield, 29% based on conversion) of N-cyclohexyldiphenylamine, and showed that about 50% of the cyclohexanone was unreacted and about 10% was converted to cyclohexanol.

H. Example 5A was repeated with 10.0 g of 20% molybdenum sulfide on alumina for 5 hours at 250-255°C and 4.1-6.6 MPa. Treatment and analysis of the reaction product as in Example 5A indicated the presence of 125 g (74% yield) of recovered diphenylamine, and 14 g (6% yield, 23% based on conversion) of N-cyclohexyldiphenylamine, and showed that about 26% of the cyclohexanone was unreacted and about 19% was converted to cyclohexanol.

## Reductive Alkylation of N,N'-Diphenyl-p-phenylenediamine with cyclohexanone

A. A mixture of 65 g (0.25 mole) of N,N'-diphenyl-p-phenylenediamine, 430 ml (4.1 mole) of cyclohexanone, and 10.0 g of 5% rhodium sulfide on carbon was added to a 1 liter MagneDrive autoclave. The vessel was sealed, purged first with nitrogen and then with hydrogen, and pressurized with hydrogen to 4.8 MPa. The reaction mixture was heated with agitation for 2.8 hours at 95-100°C and 4.1-6.2 MPa. The autoclave was cooled and depressurized. The reaction product was removed and filtered through Celite filter-aid to remove the catalyst. The solvent was removed using a rotary evaporator on a steam bath under reduced pressure. A portion of the tarry residue was separated by elution chromatography on a silica gel column into three fractions. The first fraction was equivalent to 55 g (52% yield) of white N,N'-dicyclohexyl-N,N'-diphenyl-p-phenylenediamine, melting at 147-150°C. Two recrystallizations from hexane gave a material melting at 151.5-152°C.

Anal. Calc'd for $C_{30}H_{36}N_2$:   C, 84.86; H, 8.55; N, 6.59

Found:   C, 84.85; H, 8.44; N, 6.53

The second fraction v————ivalent to 26.5 g of liquid N-cyclohexyl-N,N'-diphenyl-p-        nediamine.

Anal. Calc'd for $C_{24}$        :   C, 84.18; H, 7.65; N, 8.18

:   C, 83.73; H, 7.84; N, 8.44

The third fraction was e     alent to 10 g and was shown by tlc to be mostly N-cyclohexyl-N,   -diphenyl-p-phenylenediamine plus some recovered N,N'-diphenyl-p-phenylenediamine. The estimated yield of N-cyclohexyl-N,N'-diphenyl-p-phenylenediamine was about 40%.

B. Example on is repeated as above except that the pressure is 31-35 MPa and the product N,N'-dicyclohexyl-diamine is isolated and characterized as above.

## Example 7

### Reductive Alkylation of N,N'-Diphenyl-p-phenylenediamine with Acetone

A mixture of 65 g (0.25 mole) of N,N'-diphenyl-p-phenylenediamine, 430 ml (5.85 mole) of acetone, and 10.0 g of 5% platinum sulfide on carbon was added to a 1-liter MagneDrive autoclave. The vessel was sealed, purged first with nitrogen and then with hydrogen, and pressurized with hydrogen to 6.9 MPa. The reaction mixture was heated with agitation for 21 hours at 195-200 °C and 6.2-8.3 MPa. The autoclave was cooled and depressurized. The reaction product was removed and filtered through Celite filter-aid to remove the catalyst. Most of the solvent was removed by heating on a steam bath. The solid, white residue was washed with hexane and was then recrystallized from benzene-hexane to give N,N'-diisopropyl-N,N'-diphenyl-p-phenylenediamine, melting at 140.5-141.5°C.

Anal. Calc'd for $C_{24}H_{28}N_2$:  C, 83.68; H, 8.19; N, 8.13
Found:  C, 82.91; H, 8.31; N, 8.38

## Example 8

### Reductive Alkylation of N,N'-Diphenyl-p-phenylenediamine with Methyl Ethyl Ketone

A mixture of 65 g (0.25 mole of N,N'-diphenyl-p-phenylenediamine, 430 ml (4.8 mole) of methyl ethyl ketone, and 10.0 g of 5% platinum sulfide on carbon was added to a 1 liter MagneDrive autoclave. The vessel was sealed, purged first with nitrogen and then with hydrogen, and pressurized with hydrogen to 4.8 MPa. The reaction mixture was heated with agitation for 5.8 hours at 100-105°C and 3.5-5.5 MPa. The autoclave was cooled and depressurized. The reaction product was removed and filtered through Celite filter-aid to remove the catalyst. The solvent was removed using a rotary evaporator on a steam bath under reduced pressure.

A portion of the solid residue was separated by elution chromatography on a silica gel column into a fraction consisting of the equivalent of 55 g (59% yield) of N,N'-di-sec-butyl-N,N'-diphenyl -p-phenylenediamine, melting at 89.5-90.5°C.

Anal. Calc'd for $C_{26}H_{32}N_2$: C, 83.82; H, 8.67; N, 7.51

Found: C, 83.63; H, 8.60; N, 7.29.

A second fraction of crude N-sec-butyl-N,N'-diphenyl-p-phenylenediamine melted at 97-104°C. Recrystallization from hexane gave material melting at 105-106°C.

Anal. Calc'd for $C_{22}H_{24}N_2$: C, 83.50; H, 7.64; N, 8.85 Found: C, 82.95; H, 7.87; N, 8.94

## Example 9

### Reductive Alkylation of N,N'-Diphenyl-p-phenylenediamine with Methyl Isobutyl Ketone

A. A mixture of 65 g (0.25 mole) of N,N'-diphenyl-p-phenylenediamine, 430 ml (3.4 mole) of methyl isobutyl ketone, and 10.0 g of 5% rhodium sulfide on carbon was added to a 1-liter MagneDrive autoclave. The vessel was sealed, purged first with nitrogen and then with hydrogen, and pressurized with hydrogen to 4.8 MPa. The reaction mixture was heated with agitation for 3.3 hours at 240-250°C and 5.5-6.9 MPa. The autoclave was cooled and depressurized. The reaction product was removed and filtered through Celite filter-aid to remove the catalyst. The solvent was removed on a rotary evaporator on a steam bath at reduced pressure. The semi-solid residue was extracted with hexane and was separated into a hexane-soluble fraction and a hexane-insoluble fraction.

BAD ORIGINAL

From the hexane-soluble fraction was obtained white N-1,3-dimethylbutyl-N,N'-diphenyl-p-phenylenediamine, melting at 90.5-91°C after recrystallization from hexane.

Anal. Calc'd for $C_{24}H_{28}N_2$: C, 83.39; H, 8.33; N, 8.27 Found: C, 82.98; H, 8.13; N, 8.18.

A portion of the hexane-insoluble fraction was elution chromatographed on a silica gel column. A hexane-benzene (85:15, v/v) eluant yielded white N,N'-di-1,3-dimethylbutyl-N,N'-diphenyl-p-phenylenediamine, melting at 78.5-84.5°C. The melting point was not improved by several recrystallizations from hexane, 2-propanol-water, and methanol-water.

Anal. Calc'd for $C_{30}H_{40}H_2$: C, 84.06; H, 9.40; N, 6.53.

Found: C, 83.67; H, 9.44; N, 6.89.

B. Example 9A was repeated with 10.0 g of 5% platinum sulfide on carbon for 4.5 hours at 210-215°C and 5.5-6.9 MPa. The reaction product was filtered to remove the catalyst and the solvent was removed using a rotary evaporator as in Example 9A. Elution chromatography of a portion of the residue on a silica gel column gave the equivalent of 3.6 g (3% yield) of N,N'-di-1,3-dimethylbutyl-N,N'-diphenyl-p-phenylenediamine, identified by tlc.

## Example 10

### Reductive Alkylation of N,N'-Diphenyl-p-phenylenediamine with Methyl Isoamyl Ketone

A mixture of 65 g (0.25 mole) of N,N'-diphenyl-p-phenylenediamine, 430 ml (3.05 mole) of methyl isoamyl ketone, and 10.0 g of 5% rhodium sulfide on carbon was added to a 1-liter MagneDrive autoclave. The vessel was sealed, purged first with nitrogen and then with hydrogen, and pressurized with hydrogen to 4.8 MPa. The reaction mixture was heated with agitation for 12.7 hours at 160-165°C and 4.8-6.9 MPa. The autoclave was cooled and depressurized. The reaction product was removed and filtered through Celite filter-aid to remove the catalyst. The solvent was removed using a rotary evaporator on a steam bath under reduced pressure.

A portion of the residue was separated by elution chromatography on a silica gel column into one fraction equivalent to 57.5 g (50% yield) of liquid N,N'-di-1,4-dimethylpentyl-N,N'-diphenyl-p-phenylenediamine.

Anal. Calc'd for $C_{32}H_{44}N_2$: C, 84.15; H, 9.72; N, 6.13. Found: C, 84.24; H, 9.83; N, 6.42;

a second fraction equivalent to 32 g (36% yield) of white N-1,4-dimethylpentyl-N,N'-diphenyl-p-phenylenediamine, melting at 90.5-91°C.

Anal. Calc'd for $C_{25}H_{30}N_2$: C, 83.75; H, 8.42; N, 7.81. Found: C, 83.67; H, 8.55; N, 8.11;

and a third fraction equivalent to 1.3 g (2% yield) of recovered N,N'-diphenyl-p-phenylenediamine, identified by tlc and melting point.

## Example 11

### Reductive Alkylation of N-Phenyl-N'-2-naphthyl-p-phenylenediamine with Acetone

A mixture of 31.0 g (0.10 mole) of N-phenyl-N'-2-naphthyl-p-phenylenediamine, 260 ml ( 3.5 mole) of acetone, and 6.0 g of 5% platinum sulfide on carbon was added to a 1-liter MagneDrive autoclave. The vessel was sealed, purged first with nitrogen and then with hydrogen, and pressurized with hydrogen to 6.9 MPa. The reaction mixture was heated with agitation for 7.3 hours at 180°C and 7.3-9.7 MPa. The autoclave was cooled and depressurized. The reaction product was removed and filtered through Celite filter-aid to remove the catalyst. The solvent was removed using a rotary evaporator on a steam bath under reduced pressure. Solid N,N'-diisopropyl-N-phenyl-N'-2-naphthyl-p-phenylenediamine, melting at 123.5-124°C, was obtained by elution chromatography of a portion of the residue on a silica gel column.

Anal. Calc'd for $C_{28}H_{31}N_2$: C, 85.24; H, 7.66; N, 7.10.

Found: C, 84.96; H, 7.96; N, 7.16

## Example 12

### Reductive Alkylation of Di-2-Naphthylamine with Cyclohexanone

A mixture of 135 g (0.50 mole) of di-2-naphthylamine, 390 ml (3.75 mole) of cyclohexanone, and 10.0 g of 5% rhodium sulfide on carbon was added to a 1-liter MagneDrive autoclave. The vessel was sealed, purged first with nitrogen and then with hydrogen, and pressurized with hydrogen to 4.1 MPa. The reaction mixture was heated with agitation for 12.5 hours at 115-120°C and 3.5-5.5 MPa. The autoclave was cooled and depressurized. The reaction product was removed and filtered through Celite filter-aid to remove the catalyst. The solvent was removed from the filtrate using a rotary evaporator on a steam bath under reduced pressure. The residue was separated by elution chromatography on a silica gel column into two fractions. One fraction was equivalent to 86 g (49% yield) of white, N-cyclohexyl-di-2-naphthylamine, melting at 118-122°C. The melting point was 117-120.5 °C (mostly 117-118°C) after recrystallization from hexane, and did not change after several subsequent recrystallizations from hexane.

Anal. Calc'd for $C_{26}H_{25}N$: C, 88.85; H, 7.17; N, 3.98.

Found: C, 88.77; H, 7.19; N, 3.85.

A second fraction consisted of the equivalent of 56 g (41.5% yield) of recovered di-2-naphthylamine, identified by tlc.

Example 13

## Reductive Alkylation of N-Phenyl-N',N'-dimethyl-p-phenylenediamine with Acetone

A mixture of 50.0 g (0.236 mole) of N-phenyl-N',-N'-dimethyl-p-phenylenediamine, 175 ml (2.4 mole) of acetone and 2.3 g of 5% platinum sulfide on carbon was added to a 600-ml Magne Dash (trademark of Autoclave Engineers, Inc.) autoclave. The vessel was sealed, purged first with nitrogen and then with hydrogen, and pressurized with hydrogen to 2.8 MPa. The reaction mixture was heated with agitation for 5.2 hours at 180°C and 2.6-3.9 MPa. The autoclave was cooled and depressurized. The reaction product was removed and filtered through Celite filter-aid to remove the catalyst. The solvent was removed from the filtrate using a rotary evaporator on a steam bath under reduced pressure. Recrystallization of a portion of the residue from hexane, followed by several recrystallizations from isopropyl ether, gave N,N-dimethyl-N'-isopropyl-N'-phenyl-p-phenylenediamine melting at 71.5-72°C.

Anal. Calc'd for $C_{17}H_{22}N_2$: C, 80.26; H, 8.72; N, 11.01
Found: C, 80.49; H, 8.58; N, 10.90.

Example 14

## Reductive Alkylation of N-Phenyl-N'-methyl-N'-isopropyl-p-phenylenediamine with Acetone

A mixture of 32.0 g (0.133 mole) of N-phenyl-N'-methyl-N'-isopropyl-p-phenylenediamine, 195 ml (2.65 mole) of acetone, and 2.3 g of 5% platinum sulfide on carbon was added to a 600-ml Magne Dash autoclave. The vessel was sealed, purged first with nitrogen and then with hydrogen, and pressurized with hydrogen to 2.8 MPa. The reaction mixture was heated with agitation for 5.3 hours at 180°C and 3.6-4.1 MPa. The autoclave was cooled and depressurized. The reaction product was removed and filtered through Celite filter-aid to remove the catalyst. The solvent was removed from the filtrate using a rotary evaporator on a steam bath under reduced pressure. Elution chromatography of a portion of the residue on an alumina column gave a fraction melting at 47-49°C from which a sample of N-methyl-N'-phenyl-N,N'-diisopropyl-p-phenylenediamine melting at 48.5-49.5°C was obtained after recrystallization from hexane.

Anal. Calc'd for $C_{19}H_{26}N_2$: C, 80.80; H, 9.26; N, 9.92 Found: C, 81.25; H, 9.27; N, 10.12

## Example 15

### Reductive Alkylation of N-Phenyl-N',N'-diisopropyl-p-phenylenediamine with Acetone

A mixture of 22.0 g (0.082 mole) of N-phenyl-N',N'-diisopropyl-p-phenylenediamine, 275 ml (3.7 mole) of acetone, and 3.0 g of 5% platinum sulfide on carbon was added to a 600 ml Magne Dash autoclave. The vessel was sealed, purged first with nitrogen and then with hydrogen, and pressurized with hydrogen to 2.8 MPa. The reaction mixture was heated with agitation for 7.2 hours at 195-200°C and 3.5-4.8 MPa. The autoclave was cooled and depressurized. The reaction product was removed and filtered through Celite filter-aid to remove the catalyst. The solvent was removed from the filtrate using a rotary evaporator on a steam bath under reduced pressure. Elution chromatography of a portion of the residue on an alumina column gave a fraction from which was obtained a sample of N-phenyl-N,N',N'-triisopropyl-p-phenylenediamine melting at 61.5-63°C that gave no depression on a mixed melting point with an authentic sample prepared as described below.

A mixture of 20.0 g (0.0746 mole) of N-phenyl-N,N'-diisopropyl-p-phenylenediamine, 45 ml (0.61 mole) of acetone, and 1.3 g of 5% platinum sulfide on carbon was added to a 170-ml Magne Dash autoclave. The vessel was sealed, purged first with nitrogen and then with hydrogen, and pressurized with hydrogen to 4.8 MPa. The reaction mixture was heated with agitation for 5.7 hours at 200 °C and 5.5-5.9 MPa. The autoclave was cooled and depressurized. The reaction product was removed and filtered through Celite filter-aid to remove the catalyst. Most of the solvent was removed by distillation and the residue chromatographed on an alumina column. One fraction of a hexane eluant gave N-phenyl-N,N',N'-triisopropyl-p-phenylenediamine melting at 64-64.5°C after two recrystallizations from hexane.

<u>Anal</u>. Calc'd for $C_{21}H_{30}N_2$: C, 81.23; H, 9.74; N, 9.02.

Found: C, 80.81; H, 9.57; N, 9.18

- 22 -

0014998

We claim:

1. A method for preparing a compound having the formula A-NR-B, wherein A is phenyl, naphthyl, phenyl substituted with up to three groups, which may be the same or different and may be chlorine, bromine, linear or branched $C_1$-$C_{12}$ alkyl, or naphthyl substituted with linear or branched $C_1$-$C_{12}$ alkyl; B may be the same as or different from A and has the meanings of A, provided that B does not have more than three substituents including an amino moiety, or B is $-C_6H_4NR^1R^2$, wherein $R^1$ and $R^2$ may be the same or different and are hydrogen, provided that at least one of $R^1$ and $R^2$ is other than hydrogen, linear or branched $C_1$-$C_{18}$ alkyl, $C_5$-$C_6$ cycloalkyl, or $C_6$-$C_{10}$ aryl, provided that only one of $R^1$ and $R^2$ is naphthyl, and R is $-CHR^4R^5$ wherein $R^4$ and $R^5$ may be the same or different and are linear or branched $C_1$-$C_{18}$ alkyl or $R^4$ and $R^5$ combined are tetramethylene or pentamethylene; characterized by reacting a compound of the formula A-NH-B, wherein A and B have the meanings defined above, except that $R^1$ or $R^2$ or both may be hydrogen, with a compound having the formula $OCR^4R^5$ wherein $R^4$ and $R^5$ are as defined above; in the presence of an heterogeneous catalyst selected from metals of groups 1b, 6b, 7b, and 8 or their oxides or sulfides, and copper chromite, except for silver, gold, technetium, manganese and chromium and their oxides or sulfides, in the presence of hydrogen.

2. A method according to claim 1, characterized by the fact that A is phenyl or naphthyl; B is phenyl, naphthyl or $-C_6H_4NR^1R^2$ wherein $R^1$ is $C_1$-$C_3$ alkyl, phenyl or naphthyl, and $R^2$ is hydrogen, linear or branched $C_1$-$C_7$ alkyl or cyclohexyl; and R is $-CHR^4R^5$, wherein $R^4$ is methyl and $R^5$ is $C_1$-$C_5$ alkyl

or $R^4$ and $R^5$ combined are pentamethylene.

0014998

3.    A method according to claim 1 or 2, characterized by the fact that the catalyst is cobalt sulfide, molybdenum sulfide, nickel sulfide, palladium sulfide, platinum sulfide, rhodium sulfide or ruthenium sulfide.

4.    A method according to claim 1 characterized by the fact that the hydrogen pressure is 0.7 to 18 MPa.

5.    A method according to claim 1, characterized by the fact that the reaction temperature is 75°C to 250°C.

6.    A method according to claim 4 or 5, characterized by the fact that the catalyst is cobalt sulfide, molybdenum sulfide, nickel sulfide, palladium sulfide, platinum sulfide, rhodium sulfide or ruthenium sulfide.

European Patent Office

**EUROPEAN SEARCH REPORT**

| Category | Citation of document with indication, where appropriate, of relevant passages | Relevant to claim | CLASSIFICATION OF THE APPLICATION (Int. Cl.3) |
|---|---|---|---|
| | **DOCUMENTS CONSIDERED TO·BE RELEVANT** | | |
| X | GB - A - 1 064 958 (UNITED STATES RUBBER) <br> * claims 1 to 4 * <br> -- | 1-6 | C 07 C 85/08 <br> C 07 C 87/62 <br> C 07 C 87/58 <br> C 07 C 87/66 |
| | GB - A - 1 066 579 (ENGELHARD INDUSTRIES) <br> * page 1, lines 43 to 46, example II * <br> -- | 1,3 | B 01 J 27/04 |
| | GB - A - 1 117 332 (UNIVERSAL OIL PRODUCTS) <br> * claim 1 * <br> -- | 1-3 | **TECHNICAL FIELDS SEARCHED (Int.Cl.3)** |
| | US - A - 3 275 567 (ENGELHARD INDUSTRIES) <br> * examples II, III * <br> -- | 1,3 | B 01 J 27/04 <br> C 07 C 85/08 <br> C 07 C 87/00 |
| | DE - B - 1 293 775 (UNIROYAL) <br> * example V * <br> ---- | 1 | |

**CATEGORY OF CITED DOCUMENTS**

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

The present search report has been drawn up for all claims

| Place of search | Date of completion of the search | Examiner |
|---|---|---|
| Berlin | 19-05-1980 | FROELICH |

EPO Form 1503.1  06.78